(19) Europäisches Patentamt
European Patent Office
Office européen des brevets

(11) Publication number: **0 413 832 A1**

(12) **EUROPEAN PATENT APPLICATION published in accordance with Art. 158(3) EPC**

(21) Application number: **90904430.7**

(51) Int. Cl.[5]: **C07D 263/44**

(22) Date of filing: **09.03.90**

(86) International application number: **PCT/JP90/00316**

(87) International publication number: **WO 90/10626 (20.09.90 90/22)**

(30) Priority: **10.03.89 JP 56123/89**

(43) Date of publication of application: **27.02.91 Bulletin 91/09**

(84) Designated Contracting States: **AT BE CH DE DK ES FR GB IT LI LU NL SE**

(71) Applicant: **SAGAMI CHEMICAL RESEARCH CENTER**
**4-5, Marunouchi 1-chome**
**Chiyoda-ku Tokyo 100(JP)**

Applicant: **CHISSO CORPORATION**
**6-32, Nakanoshima 3-chome Kita-ku**
**Osaka-shi Osaka 530(JP)**

(72) Inventor: **HIRAI, Kenji 9-2-104, Minamidai 1-chome**
**Sagamihara-shi**
**Kanagawa 228(JP)**
Inventor: **FUJITA, Atsuko 9-3-401, Konakadai 5-chome**
**Chiba-shi**
**Chiba 280(JP)**
Inventor: **SATO, Hiroshi 2584 C-102, Goi**
**Ichihara-shi**
**Chiba 290(JP)**
Inventor: **HIROSE, Hiroaki 17 A-208, Tatsumidaihigashi 2-chome**
**Ichihara-shi Chiba 290(JP)**
Inventor: **YOKOTA, Masahiro**
**17 B-302, Tatsumidaihigashi 2-chome**
**Ichihara-shi Chiba 290(JP)**
Inventor: **NAGATO, Shoin**
**13-13-106, Shikahama 3-chome**
**Adachi-ku Tokyo 123(JP)**

(74) Representative: **Rauh, Peter A., Dr.**
**Vossius & Partner Siebertstrasse 4 Postfach 86 07 67**
**D-8000 München 86(DE)**

(54) **OXAZOLIDINEDIONE DERIVATIVES AND PRODUCTION THEREOF.**

(57) This invention relates to oxazolidinedione derivatives of general formula (1) and a method of producing the same, wherein $R^1$ represents a hydrogen atom or a lower alkyl group, and $R^2$ represents a hydrogen atom or a lower alkyl group as well. The above compounds serve as an intermediate for the production of benzoxazinone derivatives of general formula(II)selectively in good yields, wherein $R^1$ represents a hydrogen atom or a lower alkyl group, and $R^3$ represents a hydrogen atom or an alkyl, cyanoalkyl, alkenyl, alkynyl or aralkyl group. Said benzoxazinone derivatives are well-known and useful as a herbicide.

EP 0 413 832 A1

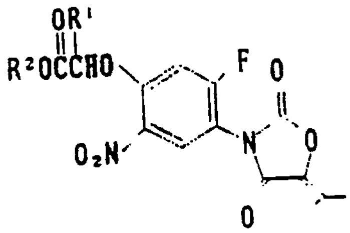

(I)

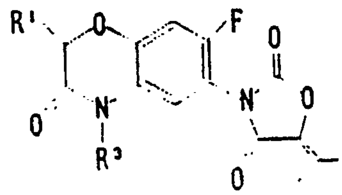

(II)

# OXAZOLIDINEDIONE DERIVATIVE AND PROCESS FOR PREPARING THE SAME

## Technical Field

This invention relates to oxazolidinedione derivatives represented by general formula (I) (hereinafter referred to as the compounds of the present invention):

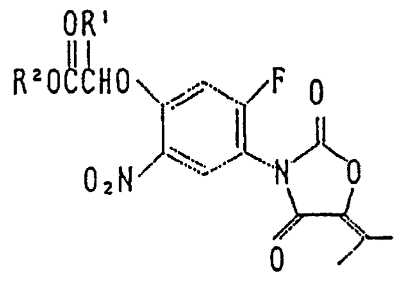

( I )

wherein $R^1$ represents a hydrogen atom or a lower alkyl group, preferably an alkyl group having 1 to 3 carbon atoms, and $R^2$ represents a hydrogen atom or a lower alkyl group, preferably an alkyl group having 1 to 6 carbon atoms and more preferably an alkyl group having 1 to 4 carbon atoms, and to a process for preparing the same.

More specifically, the present invention provides an intermediate for preparing benzoxazinone derivatives represented by general formula (III):

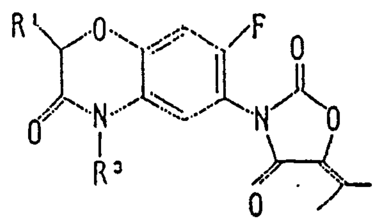

( III )

wherein $R^1$ represents a hydrogen atom or a lower alkyl group, and $R^3$ represents a hydrogen atom, an alkyl group, a cyanoalkyl group, an alkenyl group, an alkynyl group or an aralkyl group, which are useful as active ingredients of herbicides, and a process for preparing these intermediates.

## Technical Background

The compounds represented by general formula (III) are benzoxazinone derivatives having a herbicidal activity as described in the official publications of Japanese Patent Application (Kokai) No. Hei-1-308278 (EP 328001A) and Japanese Patent Application (Kokai) No. Hei-1-308211, and are useful compounds having an excellent herbicidal activity against various weeds. Accordingly, in producing the compounds represented by general formula (III), a convenient process which is capable of providing the desired compounds in good yields and good selectivity has been desired.

## Disclosure of the Invention

As a result of extensive studies on a process for preparing the compounds represented by the above general formula (III) having an excellent herbicidal activity, the present inventors found that the compounds of the present invention represented by the above general formula (I) are very important as an intermediate for preparing the same, and proved that the desired compounds represented by general formula (III) can be easily prepared from the compounds of the present invention according to the process shown below, thereby completing the present invention. That is, the compound (I) of the present invention can be

subjected to a reaction under a reductive condition thereby simultaneously effecting a reduction of the nitro group and a subsequent intramolecular condensation reaction with the carboxyl group or the ester group thereof to produce a benzoxazinone derivative represented by general formula (III'):

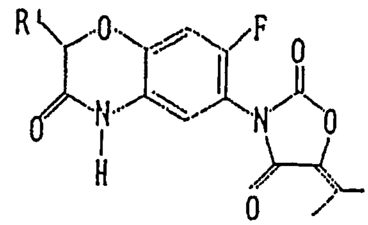

(III')

wherein $R^1$ is as defined above, and the resulting compound can be reacted with an electrophilic agent represented by general formula (IV):

$$R^{3'} - Y \qquad (IV)$$

wherein $R^{3'}$ represents an alkyl group, a cyanoalkyl group, an alkenyl group, an alkynyl group or an aralkyl group, and Y represents a cleaving group, in the presence of a base, to produce a benzoxazinone derivative represented by general formula (III"):

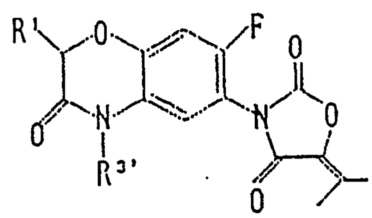

(III")

wherein $R^1$ and $R^{3'}$ are as defined above, in good yield.

The process for preparing the compounds of the present invention is described hereinafter in detail.

The compounds of the present invention can be prepared by reacting 3-(2,4-difluoro-5-nitrophenyl)-5-isopropylidene-1,3-oxazolidine-2,4-dione with a 2-hydroxycarboxylic acid or an ester thereof represented by general formula:

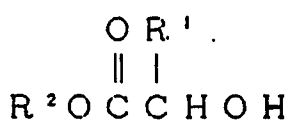

(II)

wherein $R^1$ and $R^2$ are as defined above, in the presence of a base.

The base which can be used in the above reaction includes tertiary aliphatic and aromatic amine compounds such as triethylamine, tributylamine, N-methylmorpholine, pyridine, lutidine, etc., or basic inorganic compounds such as potassium carbonate, sodium carbonate, sodium acetate, potassium acetate, etc., alkali metal alkoxides such as sodium methoxide, sodium ethoxide, potassium t-butoxide, etc., alkali metal hydroxides and alkali metal amides such as sodium hydride, potassium hydride, sodium amide, lithium amide, etc. The amount of the base used is not limited, but, generally, a catalytic amount is sufficient. The reaction can be carried out in the absence of solvents or in an appropriate organic solvent which does not affect the reaction. The organic solvent which can be used includes, for example, dimethylformamide, dimethyl sulfoxide, acetonitrile, etc. The reaction sufficiently proceeds under mild conditions near room temperature, but may be carried out under heating. Although the reaction sufficiently proceeds under atmospheric pressure, the reaction proceeds efficiently by being carried out under high pressure. After completion of the reaction, the reaction solution is poured into diluted hydrochloric acid, extracted with a solvent which is difficultly soluble in water, for example, diethyl ether, ethyl acetate, toluene or chloroform, etc., and then worked up in a usual manner whereby the desired compound can be isolated as crystals. If necessary, the compound is purified by a procedure such as silica gel column chromatography or recrystallization, etc.

The 2-hydroxycarboxylic acids or the esters thereof represented by general formula (II) as a starting material used in the above reaction are easily available or can be prepared conveniently from commercially available starting materials, and include, for example, glycolic acid, lactic acid, 2-hydroxyvaleric acid, 2-hydroxyisovaleric acid or a lower alkyl ester thereof, etc. The amount of the 2-hydroxycarboxylic acid or the ester thereof used for the reaction is not limited, and the compounds of the present invention can be obtained in good yields by using 1 to 20 molar equivalents relative to 3-(2,4-difluoro-5-nitrophenyl)-5-isopropylidene-1,3-oxazolidine-2,4-dione.

Another starting material for the above reaction, 3-(2,4-difluoro-5-nitrophenyl)-5-isopropylidene-1 ,3-oxazolidine-2,4-dione, can be prepared via the following reaction route according to the process described in the official publication of Japanese Patent Application (Kokai) No. Sho-62-174065.

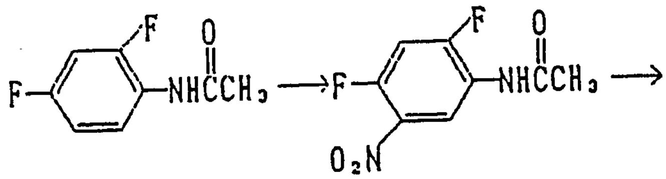

(V) (VI)

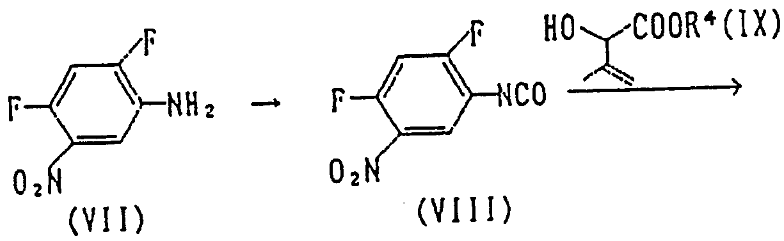

(IX)

(VII) (VIII)

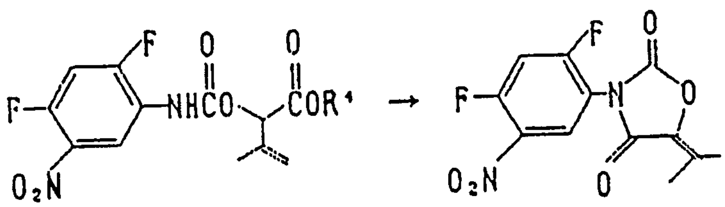

(X) (XI)

More specifically, 2,4-difluoroacetanilide (V) is nitrated using 1 to 2 molar equivalents of fuming sulfuric acid in 3 molar equivalents or more of fuming sulfuric acid at a low temperature of about -10° C to room temperature to produce 2,4-difluoro-5-nitroacetanilide (VI), and the product is subjected to a usual deacetylation reaction using a mineral acid such as hydrochloric acid or sulfuric acid, etc., in water or an alcohol solvent whereby 2,4-difluoro-5-nitroaniline (VII) can be produced.

Then, the resulting product is converted into 2,4-difluoro-5-nitrophenylisocyanate (VIII) using phosgene or a phosgene equivalent material such a phosgene dimer or trimer, etc. in an organic solvent. The amount of phosgene or the phosgene equivalent material used is not limited, and the desired isocyanate derivative (VIII) can be obtained in high yields by using 1 to 10 molar equivalents as phosgene relative to the aniline derivative (VII). Any organic solvent which does not adversely affect the reaction can be used, and, for example, ethyl acetate, chloroform, acetonitrile, acetone, N,N-dimethylformamide, etc. can be used. The reaction can be carried out by adding dropwise the aniline derivative slowly to a solution of phosgene or the phosgene equivalent material at room temperature and reacting them under conditions of the refluxing temperature of the reaction solvent to about 100° C whereby the isocyanate derivative can be synthesized. The isocyanate derivative (VIII) thus obtained is reacted with a 2-hydroxy-3-methyl-3-butenoic acid ester in an organic solvent in the presence of a base to Produce a carbamic acid ester (X). The reaction can be carried out under heating, but it proceeds sufficiently at room temperature whereby the desired carbamic

acid ester (X) can be obtained in good yields. The organic solvent which can be used includes ethers such as diethyl ether, tetrahydrofuran, dioxane, dimethoxyethane, etc., aromatic solvents such as ethyl acetate, acetonitrile, N,N-dimethylformamide, dimethyl sulfoxide, etc. The base which can be used includes amines such as triethylamine, tributylamine, N-methylmorpholine, N,N-dimethylaniline, N,N-diethylaniline, pyridine, lutidine, etc., and alkali metal salts such as potassium carbonate, sodium carbonate, sodium methoxide, potassium t-butoxide, etc., and the amount of the base used is not particularly limited, with a catalytic amount being sufficient. Also, the desired carbamic acid ester (X) can be obtained in good yields by using at least one molar equivalent of the 2-hydroxy-3-methyl-3-butenoic acid ester relative to the isocyanate (VII).

The carbamic acid ester (X) thus obtained can be treated with a base to obtain the oxazolidinedione derivative (XI) as a starting material for preparing the compounds of the present invention, i.e., 3-(2,4-difluoro-5-nitrophenyl)-5-isopropylidene-1,3-oxazolidine-2,4-dione. The reaction is carried out in a usual organic solvent at a temperature of from room temperature to a refluxing temperature of the solvent, and the organic solvent which can be used includes ethers such as diethyl ether, tetrahydrofuran, dioxane, dimethoxyethane, etc., aromatic solvents such as benzene, toluene, etc., or ethyl acetate, acetonitrile, N,N-dimethylformamide, dimethyl sulfoxide, etc. The base which can be used includes amines such as triethylamine, tributylamine, N-methylmorpholine, N,N-dimethylaniline, N,N-diethylaniline, pyridine, lutidine, etc. and alkali metal salts such as potassium carbonate, sodium carbonate, sodium methoxide, potassium t-butoxide, etc. The amount of the base used is not particularly limited, with a catalytic amount being sufficient. In particular, acetonitrile as an organic solvent and potassium carbonate as a base are preferred in view of that the desired oxazolidinedione derivative (XI) can be obtained in good yields under mild conditions.

Further, 3-(2,4-difluoro-5-nitrophenyl)-5-isopropylidene-1,3-oxazolidine-2,4-dione (XI) can be directly prepared by reacting the isocyanate derivative (VIII) with the 2-hydroxy-3-methyl-3-butenoic acid ester (IX) without isolating the carbamic acid ester (X).

The present invention is described hereinafter in detail by referring to Examples and Reference Examples.

(Examples)

Preparation examples of the compounds of the present invention are described below.

Example 1

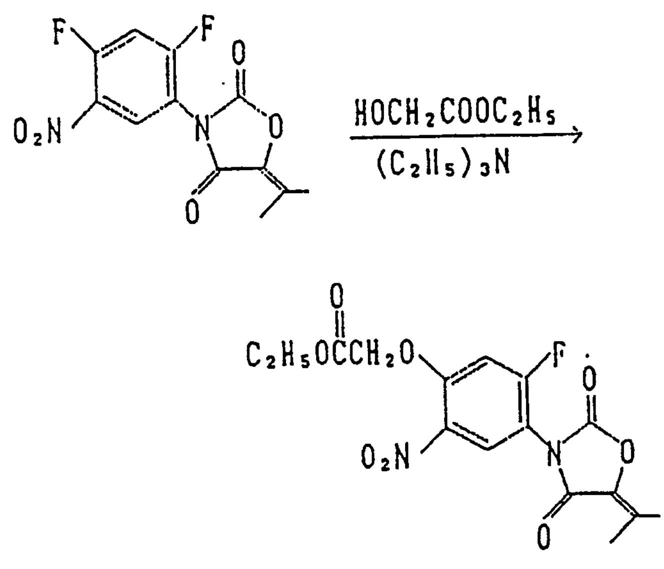


3-(2,4-Difluoro-5-nitrophenyl)-5-isopropylidene-1,3-oxazolidine-2,4-dione (4.1 g, 13.6 mmol) and ethyl glycolate (20 ml) were placed in a 100 cc round bottom flask, and triethylamine (4 ml) was dropwise added

thereto while stirring at room temperature. After stirring overnight at room temperature, 1N hydrochloric acid was added to the reaction mixture. The mixture was extracted with ethyl acetate (20 ml x 3), and the organic layer was washed with water (10 ml x 3). The resulting ethyl acetate solution was dried over anhydrous magnesium sulfate. The drying agent was filtered off, and the solvent was distilled off under reduced pressure to obtain a yellow oily substance (6.5 g). The product was isolated and purified by silica gel column chromatography to obtain a white solid (3.56 g) which was confirmed to be 3-(2-fluoro-4-ethoxycarbonylmethyloxy-5-nitrophenyl)-5-isopropylidene-1,3-oxazolidine-2,4-dione from the results of analysis on $^1$H-NMR and IR spectra, etc.

Melting Point: 130 - 135 °C.

$^1$H-NMR Spectrum ( $CDCl_3$):

δ 1.28(3H,t,J = 7.5Hz),2.03(3H,s),2.26(3H,s),4.25(2H,q J = 7.5Hz),4.78(2H,s),6.88(1H,d,JHF = 11.1Hz),8.99-(1H,d, JHF = 7.5Hz)ppm

IR Spectrum (KBr disk):

1820, 1740, 1685 $cm^{-1}$.

Example 2

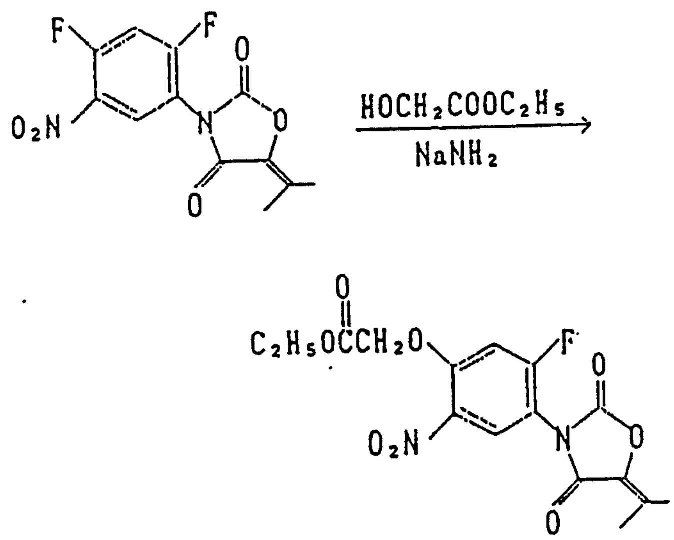


3-(2,4-Difluoro-5-nitrophenyl)-5-isopropylidene-1,3-oxazolidine-2,4-dione (298 g, 1.0 mol), ethyl glycolate (104 g, 1.0 mol) and dioxane (500 ml) as a solvent were placed in a 1,000 cc round bottom flask, and sodium amide (39g, 1.0 mol) was added thereto slowly while cooling in an ice bath. After generation of ammonia gas ceased, 1N hydrochloric acid (500 ml) was added to the reaction mixture, and the mixture was extracted with ethyl acetate (300 ml x 3). The organic layer was washed with water (100 ml x 3), and dried over anhydrous magnesium sulfate. After removing the drying agent by filtration, the solvent was distilled off under reduced pressure, and the resulting solid was recrystallized from ether hexane to obtain 3-(2-fluoro-4-ethoxycarbonylmethyloxy-5-nitrophenyl)-5-isopropylidene-1,3-oxazolidine-2,4-dione (344 g, 0.9 mol) as a substantially pure product in a yield of 90%.

Example 3

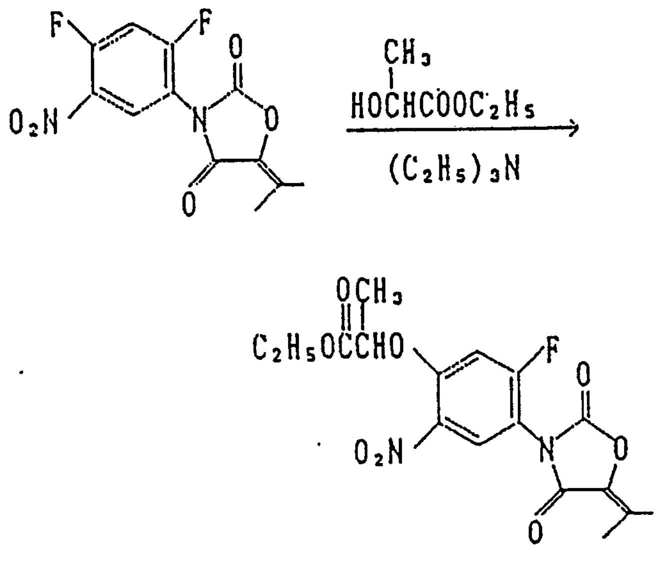


3-(2,4-Difluoro-5-nitrophenyl)-5-isopropylidene-1,3-oxazolidine-2,4-dione (4.1 g, 13.6 mmol) and ethyl (-)-lactate (15 ml) were placed in a 100 cc round bottom flask, and triethylamine (4 ml) was added dropwise thereto while stirring at room temperature. After stirring overnight at room temperature, 1N hydrochloric acid was added to the reaction mixture. The mixture was extracted with ethyl acetate (10 ml x 3), and the organic layer was washed with water (5 ml x 3). The resulting ethyl acetate solution was dried over anhydrous magnesium sulfate to obtain a yellow oily substance (5.7 g). The product was recrystallized from ethyl acetate/hexane to obtain a yellow transparent solid (1.16 g) which was confirmed to be (+)-3-[2-fluoro-4-(1-ethoxycarbonyl)ethyloxy-5-nitrophenyl]-5-isopropylidene-1,3-oxazolidine-2,4-dione from the results of analysis on $^1$H-NMR and IR spectra, etc.

Melting Point: 148 - 149 ° C.

$^1$H-NMR spectrum ( $CDCl_3$):

δ1.27(3H,t,J = 6.6Hz),1.71(3H,d,J = 6.3Hz),2.06(3H,s), 2.28(3H,s),4.23(2H,q,J = 6.6Hz),4.82(1H,q,J = 6.3Hz), 6.82(1H,d,JHF = 11.1Hz),8.31(1H,d,JHF = 8.1Hz)ppm.

IR Spectrum (KBr disk):

1820, 1745, 1685 $cm^{-1}$.

Example 4

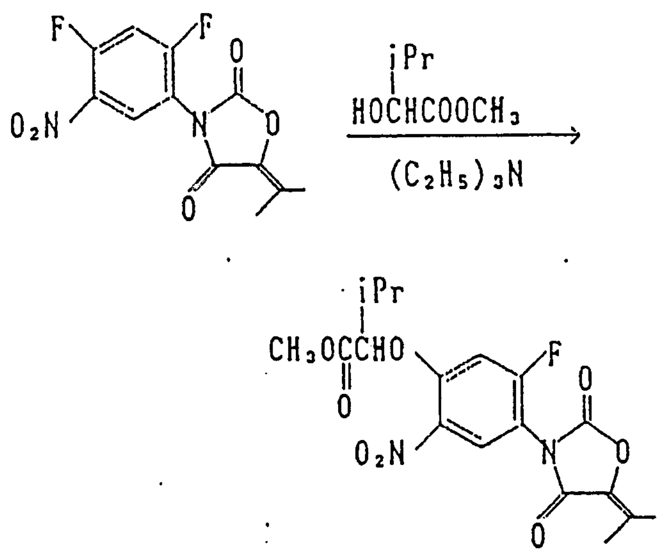


(wherein iPr represents an isopropyl group)

A mixture of 3-(2,4-difluoro-5-nitrophenyl)-5-isopropylidene-1,3-oxazolidine-2,4-dione (298 mg, 1.0

mmol), methyl 2-hydroxyisovalenate (1.0 g, 7.6 mmol) and triethylamine was reacted overnight at 2,500 atms. at room temperature using a high pressure reaction apparatus. After completion of the reaction, 0.1N hydrochloric acid (50 ml) was added to the mixture, and the mixture was extracted with ethyl acetate (25 ml x 2). The organic layer was washed with water (20 ml x 5) and dried over anhydrous magnesium sulfate. The drying agent was filtered off, the solvent was distilled off under reduced pressure to obtain a pale yellow oily substance. The product was then isolated and purified using silica gel column chromatography to obtain a colorless oily substance (319 mg) which was confirmed to be 3-[2-fluoro-4-(1-methoxycarbonyl)-isobutyloxy-5-nitrophenyl]-5-isopropylidene-1,3-oxazolidine-2,4-dione from the results of analysis on $^1$H-NMR and IR spectra, etc.

$^1$H-NMR spectrum ( $CDCl_3$):
δ1.10(6H,d,J = 7.0Hz),2.05(3H,s),2.27(3H,s),2.40(1H,m), 3.78(3H,s),4.52(1H,d,J = 4.0Hz),6.73-(1H,d,JHF = 11.0Hz). 8.03(1H,d,JHF = 7.0Hz)ppm.
IR spectrum (KBr disk):
1820, 1740, 1685 $cm^{-1}$.

Example 5

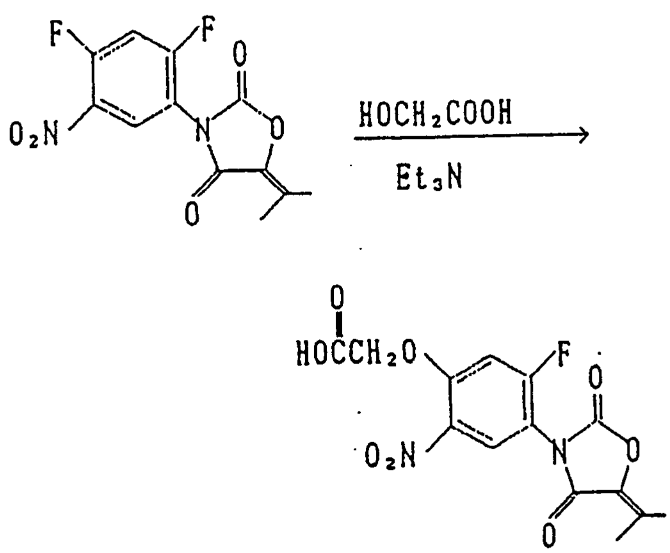


3-(2,4-Difluoro-5-nitrophenyl)-5-isopropylidene-1,3-oxazolidine-2,4-dione (304 mg, 1.02 mmol) and glycolic acid (203 mg, 2.67 mmol) were placed in a 50 cc round bottom flask, and melted by heating on an oil bath at 100 °C. Then, triethylamine (0.5 ml) was added dropwise thereto, followed by stirring at 100 °C for an additional one hour. To the reaction mixture were added 1N hydrochloric acid (25 ml) and water (25 ml), and the mixture was extracted with ethyl acetate (25 ml x 3). The organic layer was washed with water (25 ml x 3) and dried over anhydrous magnesium sulfate. The drying agent was filtered off, and the solvent was distilled off under reduced pressure to obtain a yellow oily substance. The resulting oily substance was isolated and purified by using silica gel column chromatography to obtain 3-(2-fluoro-4-hydroxycarbonyl-methyloxy-5-nitrophenyl)-5-isopropylidene-1,3-oxazolidine-2,4-dione.

Melting point: 105 - 110 °C.
$^1$H-NMR spectrum ( $CDCl_3$ - $CD_3OD$ ):
δ2.03(3H,s),2.30(3H,s),4.88(2H,s),6.97(1H,d,JHF = 10.0 Hz),8.07(1H,d,JHF = 7.0Hz)ppm.
IR Spectrum (KBr disk):
1820, 1740, 1680 $cm^{-1}$.

Next, preparation examples of 3-(2,4-difluoro-5-nitrophenyl)-5-isopropylidene-1,3-oxazolidine-2,4-dione as a starting material for the preparation of the compounds of the present invention are shown in Reference Examples 1 to 5.

Reference Example 1

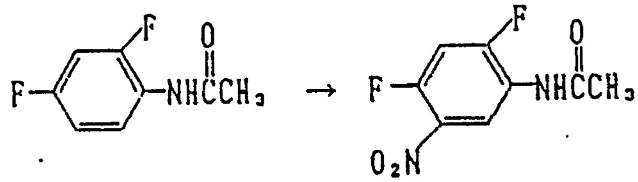

25% Fuming sulfuric acid (25 ml) was placed in a 100 cc three-necked flask equipped with a stirrer, and 2,4-difluoroacetanilide (10.0 g, 58.4 mmol) was added thereto in small portions while vigorously stirring under ice-cooling to prepare a suspension. Subsequently, fuming nitric acid (3 ml) was then added dropwise thereto over a period of 30 minutes while vigorously stirring under ice-cooling. After completion of the dropwise addition, the reaction mixture was stirred for 2 hours under ice-cooling and further for 1 hour at room temperature and added to ice-water while stirring. The precipitated solid was collected by filtration, washed with water and dried under reduced pressure to obtain a white solid which was confirmed to be 2,4-difluoro-5-nitroacetanilide (10.0 g, 79%) by $^1$H-NMR and IR spectra.

Melting point: 149 - 150 °C.

$^1$H-NMR Spectrum ( $CDCl_3$):

δ2.23(3H,s),7.11(1H,dd,JHF = 10.5,10.5Hz),9.03(1H,dd, JHF = 8.7,8.7Hz),9.35(1H,br s)ppm.

IR Spectrum (KBr disk):

3020-3240, 1675, 1610 $cm^{-1}$.

Reference Example 2

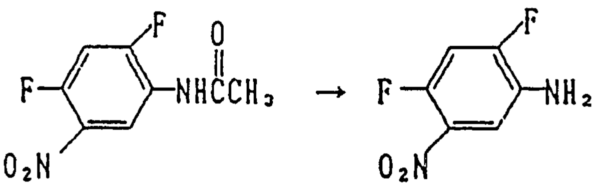

2,4-Difluoro-5-nitroacetanilide (10.0 g, 46.3 mmol), methanol (120 ml) and 2N hydrochloric acid (120 ml) were placed in a 300 cc round bottom flask and stirred for 2 hours under refluxing. After completion of the reaction, methanol was distilled off under reduced pressure. The resulting solution was rendered basic by adding sodium bicarbonate and extracted with ethyl acetate (100 ml x 3). The organic layer was washed with water (50 ml x 3) and dried over anhydrous magnesium sulfate. After removing the drying agent, the filtrate was concentrated under reduced pressure. The resulting yellow solid was confirmed to be 2,4-difluoro-5-nitroaniline (7.3 g, 91%).

Melting point: 97 - 100 °C.

$^1$H-NMR Spectrum ( $CDCl_3$):

δ3.79(2H,br s),6.96(1H,dd,JHF = 11.1,11.1Hz),7.49(1H,dd, JHF = 8.4,8.4Hz)ppm.

IR Spectrum (KBr disk):

3420, 3350, 3230, 1600 $cm^{-1}$.

Reference Example 3

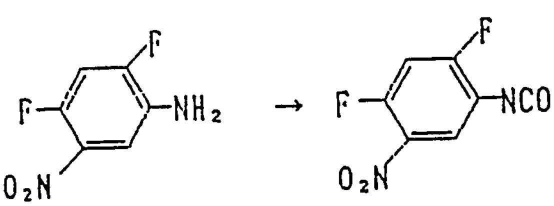

Trichloromethyl chloroformate (phosgene dimer; 10.4 ml, 86.2 mmol) and ethyl acetate (150 ml) were

placed in a 500 cc round bottom flask, and a solution of 2,4-difluoro-5-nitroaniline (10.0 g, 57.4 mmol) in ethyl acetate (150 ml) was added dropwise thereto over a period of 1.5 hour while stirring at room temperature. After completion of dropwise addition, the solvent and any excess of the phosgene dimer, etc. were removed by heating at 90° C to obtain a pale brown oily substance which was confirmed to be substantially pure 2,4-difluoro-5-nitrophenylisocyanate (11.0 g, 96%) by $^1$H-NMR and IR spectra.

$^1$H-NMR Spectrum ( $CDCl_3$):
δ7.18(1H,dd,JHF = 9.6,9.6Hz),7.92(1H,dd,JHF = 7.8,7.8Hz) ppm.
IR Spectrum (KBr disk): 2250 $cm^{-1}$.

Reference Example 4

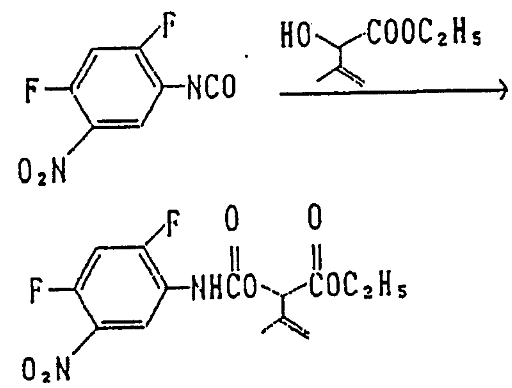

2,4-Difluoro-5-nitrophenylisocyanate (11.0 g, 48.6 mmol) and ethyl 2-hydroxy-3-methyl-3-butenoate (7.7 g, 53.4 mmol) were placed in a 300 cc round bottom flask and dissolved in diethyl ether (200 ml). Triethylamine (7.4 ml, 53.1 mmol) was added dropwise thereto at room temperature, and, after completion of the dropwise addition, the mixture was stirred for an additional one hour. After completion of the reaction, insoluble materials were filtered off, and the filtrate was washed with 1N hydrochloric acid. The ether layer was dried over anhydrous magnesium sulfate and, thereafter, concentrated under reduced pressure to obtain a yellow solid. This substance was purified by silica gel column chromatography (eluent: ethyl acetate/hexane = 1/2) to obtain a white solid which was confirmed to be ethyl 2-[N-(2,4-difluoro-5-nitrophenyl)carbamoyloxy]-3-methyl-3-butenoate (14.5 g, 90%).

Melting point: 90 - 92° C.
$^1$H-NMR Spectrum ( $CDCl_3$):
δ1.63(3H,t,J = 6.6Hz),1.86(3H,s),4.26(2H,q,J = 6.6Hz), 5.16(1H,m),5.26(1H,s),5.49(1H,s),7.09-(1H,dd,JHF = 9.3, 9.3Hz),7.13(1H,br s),8.92(1H,dd,JHF = 9.0,9.0Hz)ppm.
IR Spectrum (KBr disk):
3330, 1750, 1725 $cm^{-1}$.

Reference Example 5

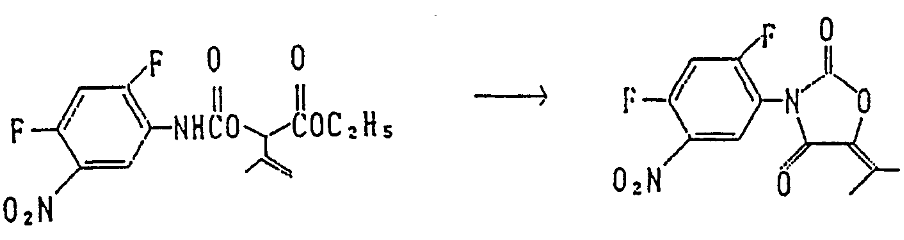

Ethyl 2-[N-(2,4-difluoro-5-nitrophenyl)carbamoyloxy]-3-methyl-3-butenoate (20.0 g, 58.1 mmol) was placed in a 500 cc round bottom flask and dissolved in acetonitrile (400 ml). To this solution was added anhydrous potassium carbonate (8 g), followed by stirring at room temperature for one hour. After completion of the reaction, the solution was rendered acidic by adding 1N hydrochloric acid and extracted

with ethyl acetate (700 ml). The organic layer was washed with water and then dried over anhydrous magnesium sulfate. The drying agent was removed, and the solution was concentrated under reduced pressure to obtain a yellow oily substance. Then, methanol was added thereto, and the pale yellow solid precipitated by cooling was collected by filtration. The resulting solid was confirmed to be 3-(2,4-difluoro-5-nitrophenyl)-5-isopropylidene-1,3-oxazolidine-2,4-dione (15.3 g, 88%).

Melting Point: 120 - 121 °C.

$^1$H-NMR Spectrum ( $CDCl_3$):

δ2.07(3H,s),2.30(3H,s),7.26(1H,dd,JHF = 9.9,9.9Hz),7.13 (1H,br s),8.24(1H,dd,JHF = 9.0,9.0Hz)ppm.

IR Spectrum (KBr disk):

1815, 1745, 1685 $cm^{-1}$.

In the following, preparation examples of the compounds having a herbicidal activity represented by the above-described general formula (III) from the compounds of the present invention are shown in Reference Examples 6 to 12.

Reference Example 6

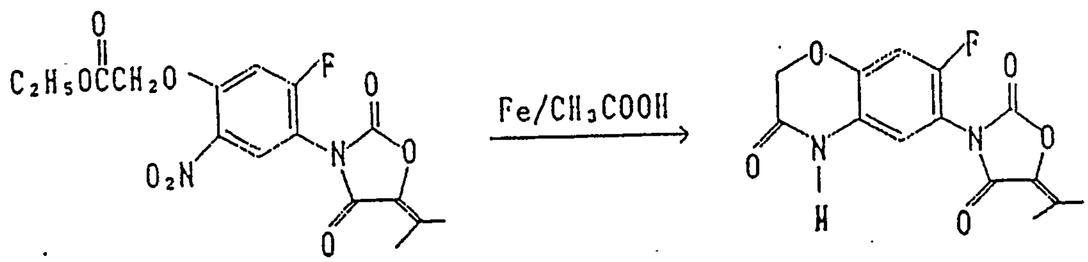


Reduced iron (6.8 g) was placed in a 300 cc three-necked flask equipped with a stirrer, a dropping funnel and a Dimroth condenser, and then acetic acid (20 ml) was added thereto, followed by heat-refluxing until the mixture became a white suspension. Then, an ethyl acetate (10 ml) solution of 3-(2-fluoro-4-ethoxycarbonylmethyloxy-5-nitrophenyl)-5-isopropylidene-1,3-oxazolidine-2,4-dione (2.76 g, 7.2 mmol) was added dropwise thereto under refluxing. After completion of dropwise addition, the mixture was stirred for one hour under refluxing and, after cooling, insoluble materials in the system were filtered off. 1N hydrochloric acid (50 ml) was added to the resulting solution, and the mixture was extracted with ethyl acetate (50 ml x 3). The organic layer was washed with water (10 ml x 3) and dried over anhydrous magnesium sulfate. The drying agent was removed, and the solution was distilled off to obtain a pale brown solid (2.1 g). The product was confirmed to be 3-(7-fluoro-2H-1,4-benzoxazine-3(4H)-one-6-yl)-5-isopropylidene-1,3-oxazolidine-2,4-dione from the results of analysis on $^1$H-NMR and IR spectra, etc.

Melting Point: 222 - 223 °C.

$^1$H-NMR Spectrum ( $CDCl_3$-DMSO-$d_6$):

δ2.03(3H,s),2.26(3H,s),4.56(2H,s),6.81(1H,d,JHF = 10.5 Hz),6.90(1H,d,JHF = 6.0Hz),7.43(1H,br)ppm.

IR Spectrum (KBr disk):

1815, 1745, 1695 $cm^{-1}$.

Reference Example 7

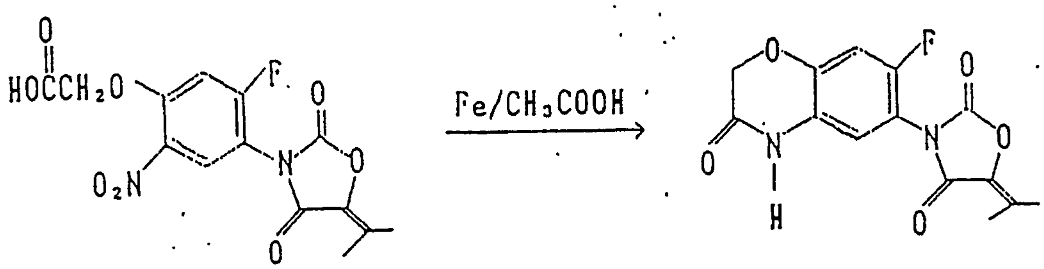

Reduced iron (1.4 g) was placed in a 50 cc three-necked flask equipped with a stirrer, a dropping funnel and a Dimroth condenser, and then acetic acid (10 ml) was added thereto, followed by heat-refluxing until the mixture became a white suspension. Then, ethyl acetate (10 ml) solution of 3-(2-fluoro-4-hydroxycarbonylmethyloxy-5-nitrophenyl)-5-isopropylidene-1,3-oxazolidine-2,4-dione (168 mg) was added dropwise thereto under refluxing. After competion of the dropwise addition, the mixture was stirred for 3 hours at 80° C and, after cooling, insoluble materials in the system were filtered off. Water (50 ml) was added to the resulting solution, and the mixture was extracted with ethyl acetate (20 ml x 3). The organic layer was washed with water (20 ml) and dried over anhydrous magnesium sulfate. The drying agent was removed, and the solution was distilled off to obtain a pale brown solid (80 mg). The product was confirmed to be 3-(7-fluoro-2H-1,4-benzoxazine-3(4H)-one-6-yl)-5-isopropylidene-1,3-oxazolidine-2,4-dione from the results of analysis on $^1$H-NMR and IR spectra, etc. The melting point, $^1$H-NMR and IR spectra were the same as those described in Reference Example 6.

## Reference Example 8

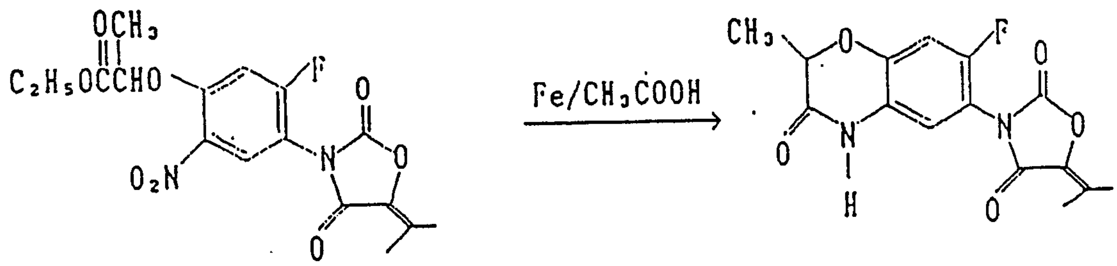


Reduced iron (3,8 g ) was placed in a 300 cc three-necked flask equipped with a stirrer, a dropping funnel and a Dimroth condenser, and then acetic acid (30 ml) was added thereto, followed by heat-refluxing until the mixture became a white suspension. Then, an ethyl acetate (20 ml) solution of (+)-3-[2-fluoro-4-(1-ethoxycarbonyl)ethyloxy-5-nitrophenyl]-5-isopropylidene-1,3-oxazolidine-2,4-dione (1.1 g, 2.8 mmol) was added dropwise thereto under refluxing. After completion of the dropwise addition, the mixture was stirred for one hour under refluxing and, after cooling, insoluble materials in the system were filtered off. 1N hydrochloric acid (50 ml) was added to the resulting solution, and the mixture was extracted with ethyl acetate (50 ml x 3). The organic layer was washed with water (10 ml x 3) and dried over anhydrous magnesium sulfate. The drying agent was removed, and the solution was distilled off to obtain a pale brown solid (0.91 g). The product was confirmed to be (+)-3-(7-fluoro-2-methyl-2H-1,4-benzoxazine-3(4H)-one-6-yl)-5-isopropylidene-1,3-oxazolidine-2,4-dione from the results of analysis on $^1$H-NMR and IR spectra, etc.

Melting point: 219 - 220° C.

Specific Rotation $[\alpha]_D$ = 8.21 (20° C, c=1.12, acetone).

$^1$H-NMR Spectrum ( $CDCl_3$):

δ1.59(3H,d,J=6.3Hz),2.04(3H,s),2.27(3H,s),4.68(1H,q, J=6.3Hz),6.78(1H,d,JHF=6.3Hz),6.85-(1H,d,JHF=10.5Hz), 9.20(1H,br)ppm.

IR Spectrum (KBr disk):

1815, 1745, 1690 $cm^{-1}$.

## Reference Example 9

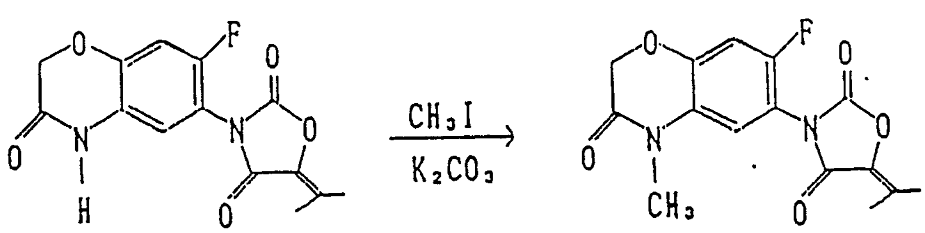

3-(7-Fluoro-2H-1,4-benzoxazine-3(4H)-one-6-yl)-5-isopropylidene-1,3-oxazolidine-2,4-dione (139 g, 0.42 mmol) and potassium carbonate (130 mg) were placed in a 25 cc round bottom flask and dissolved in N,N-dimethylformamide (5 ml). Then, methyl iodide (500 μℓ) was added dropwise to the solution while stirring at room temperature for additional 3 hours. After completion of the reaction, IN hydrochloric acid was added thereto, and the mixture was extracted with ethyl acetate (2 ml x 3). The organic layer was washed with water (1 ml x 3) and dried over anhydrous magnesium sulfate. The drying agent was removed, and the resulting solution was concentrated under reduced pressure to obtain a brown oily substance (250 mg). The product was isolated and purified by silica gel column chromatography to obtain a white solid (123 mg). The product was confirmed to be 3-(7-fluoro-4-methyl-2H-1,4-benzoxazine-3(4H)-one-6-yl)-5-isopropylidene-1,3-oxazolidine-2,4-dione from the results of analysis on $^1$H-NMR and IR spectra, etc.

$^1$H-NMR Spectrum ( $CDCl_3$):

δ2.05(3H,s),2.28(3H,s),3.32(3H,s),4.65(2H,s),6.86(1H, d,JHF = 6.9Hz),6.89(1H,d,JHF = 10.5Hz)ppm.

IR Spectrum (KBr disk):

1825, 1730, 1690 $cm^{-1}$.

Reference Example 10

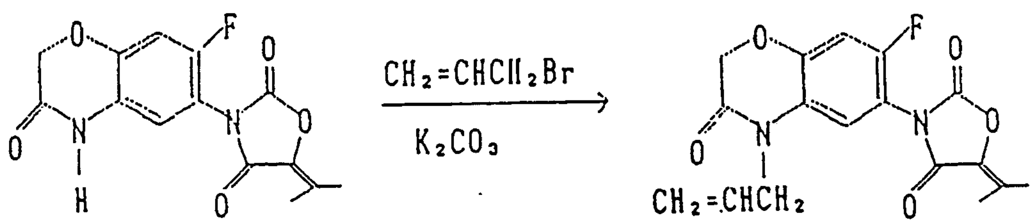


3-(7-Fluoro-2H-1,4-benzoxazine-3(4H)-one-6-yl)-5-isopropylidene-1,3-oxazolidine-2,4-dione (200 mg, 0.64 mmol) and potassium carbonate (200 mg) were placed in a 25 cc round bottom flask and dissolved in N,N-dimethylformamide (5 ml). Then, allyl iodide (500μℓ) was added dropwise to the solution while stirring at room temperature, followed by stirring at room temperature for an additional 4 hours. After completion of the reaction, 1N hydrochloric acid was added thereto, and the mixture was extracted with ethyl acetate (2 ml x 3). The organic layer was washed with water (1 ml x 3) and dried over anhydrous magnesium sulfate. The drying agent was removed, and the resulting solution was concentrated under reduced pressure to obtain a brown oily substance (138 mg). The product was isolated and purified by silica gel column chromatography to obtain a white solid (97 mg). The product was confirmed to be 3-(7-fluoro-4-allyl-2H-1,4-benzoxazine-3(4H)-one-6-yl)-5-isopropylidene-1,3-oxazolidine-2,4-dione from the results of analysis on $^1$H-NMR and IR spectra, etc.

Melting point: 166 - 168° C.

$^1$H-NMR Spectrum ( $CDCl_3$):

δ2.04(3H,s),2.27(3H,s),4.51(2H,ddd,J = 1.5,1.5,5.1Hz), 5.19(1H,ddt,J = 1,4,16.2,1.5Hz),5.23-(1H,ddt,J = 1.4,7.8, 1.5Hz),5.82(1H,ddt,J = 16.2,2.7,8.5Hz),6.85(1H,d,JHF = 6.9Hz),6.88-(1H,d,JHF = 10.2Hz)ppm.

IR Spectrum (KBr disk):

1810, 1740, 1690 $cm^{-1}$.

Reference Example 11

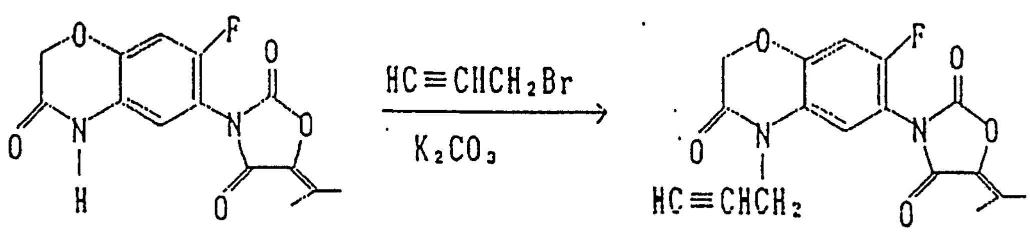

3-(7-Fluoro-2H-1,4-benzoxazine-3(4)-one-6-yl)-5-isopropylidene-1,3-oxazolidine-2,4-dione (347 mg, 0.03 mmol) and potassium carbonate (100 mg) were placed in a 25 cc round bottom flask and dissolved in N,N-dimethylformamide (30 ml). Then, propargyl bromide (200$\mu\ell$) was added dropwise to the solution while stirring at room temperature for an additional 5 hours. After completion of the reaction, 1N hydrochloric acid was added thereto, the mixture was allowed to stand at room temperature, and the precipitated crystals were isolated by filtration (345 mg). This product was confirmed to be 3-(7-fluoro-4-propargyl-2H-1,4-benzoxazine-3(4)-one-6-yl)-5-isopropylidene-1,3-oxazolidine-2,4-dione from the results of analysis on $^1$H-NMR and IR, etc.

Melting Point: 194 - 195 °C.

$^1$H-NMR spectrum ( $CDCl_3$):

δ2.03(3H,s),2.23(1H,t,J=2.1Hz),2.27(3H,s),4.63(2H,d,J =2.1Hz),4.65(2H,s),6.90(1H,d,JHF=9.6Hz),7.09-(1H,d, JHF=6.3Hz)ppm.

IR Spectrum (KBr disk):

1810, 1740, 1690 $cm^{-1}$.

Reference Example 12

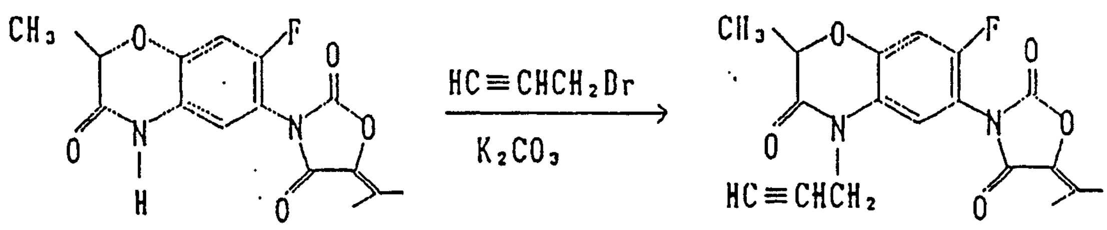


(+)-3-(7-Fluoro-2-methyl-2H-1,4-benzoxazine-3(4)-one-6-yl)-5-isopropylidene-1,3-oxazolidine-2,4-dione (163 mg, 0.51 mmol) and potassium carbonate (140 mg) were placed in a 25 cc round bottom flask and dissolved in N,N-dimethylformamide (5 ml). Then, propargyl bromide (300 $\mu\ell$) was added dropwise to the solution while stirring the mixture at room temperature, followed by stirring at room temperature for an additional 5 hours. After completion of the reaction, 1N hydrochloric acid was added thereto, the mixture was allowed to stand at room temperature., and the precipitated crystals were isolated by filtration (167 mg). This product was confirmed to be 3-(7-fluoro-2-methyl-4-propargyl-2H-1,4-benzoxazine-3(4)-one-6-yl)-5-isopropylidene-1,3-oxazolidine-2,4-dione from the results of analysis on $^1$H-NMR and IR, etc.

Melting point: 186 - 190 °C.

Specific Rotation $[\alpha]_D$ = 17.00 (20 °C, c=0.80, acetone).

$^1$H-NMR Spectrum ( $CDCl_3$):

δ1.57(3H,d,J=6.6Hz),2.03(3H,s),2.25(1H,t,J=1.5Hz), 2.27(3H,s),4.63(2H,d,J=1.5Hz),4.68(1H,d,J=6.6Hz), 6.89(1H,d,JHF=9.9Hz),7.05(1H,d,JHF=6.5Hz)ppm.

IR Spectrum (KBr disk):

1810, 1745, 1695 $cm^{-1}$.

## Claims

1. An oxazolidinedione derivative represented by general formula.

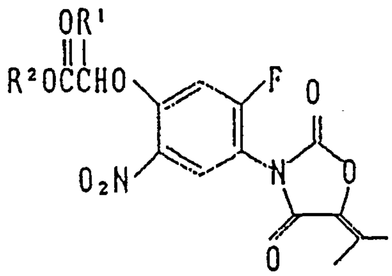

(I)

wherein $R^1$ represents a hydrogen atom or a lower alkyl group, and $R^2$ represents a hydrogen atom or a lower alkyl group.

2. An oxazolidinedione derivative as claimed in claim 1, wehrein $R^1$ is a hydrogen atom or an alkyl group having 1 to 3 carbon atoms, and $R^2$ is a hydrogen atom or an alkyl group having 1 to 6 carbon atoms.

3. An oxazolidinedione derivative as claimed in claim 2, wherein $R^2$ is a hydrogen atom or an alkyl group having 1 to 4 carbon atoms.

4. A process for preparing an oxazolidinedione derivative represented by general formula:

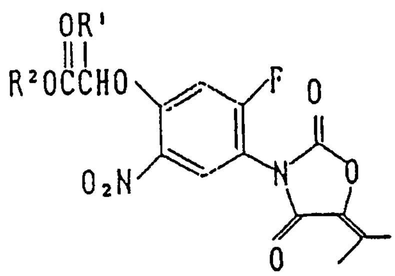

(I)

wherein $R^1$ represents a hydrogen atom or a lower alkyl group, and $R^2$ represents a hydrogen atom or a lower alkyl group, characterized by reacting 3-(2,4-difluoro-5-nitrophenyl)-5-isopropylidene-1,3-oxazolidine-2,4-dione with a 2-hydroxycarboxylic acid or an ester thereof represented by general formula:

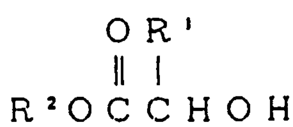

(II)

wherein $R^1$ and $R^2$ are as defined above, in the presence of a base.

5. A process as claimed in claim 4, wherein $R^1$ is a hydrogen atom or an alkyl group having 1 to 3 carbon atoms, and $R^2$ is a hydrogen atom or an alkyl group having 1 to 6 carbon atoms.

# INTERNATIONAL SEARCH REPORT

International Application No PCT/JP90/00316

**I. CLASSIFICATION OF SUBJECT MATTER** (if several classification symbols apply, indicate all) [8]

According to International Patent Classification (IPC) or to both National Classification and IPC

Int. $Cl^5$ C07D263/44

**II. FIELDS SEARCHED**

Minimum Documentation Searched [7]

| Classification System | Classification Symbols |
|---|---|
| IPC | C07D263/44, 265/36, 413/04 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| T | EP, A2, 338533 (Sumitomo Chemical Co., Ltd.), 25 October 1989 (25. 10. 89) | 1 - 5 |
| P | EP, A1, 328001 (Sagami Chemical Research Center et al.), 16 August 1989 (16. 08. 89) | 1 - 5 |
| A | EP, A1, 304935 (Sumitomo Chemical Co., Ltd.), 1 March 1989 (01. 03. 89) | 1 - 5 |
| A | JP, A, 61-140570 (Sumitomo Chemical Co., Ltd.), 27 June 1986 (27. 06. 86), (Family: none) | 1 - 5 |
| A | JP, A, 61-140573 (Sumitomo Chemical Co., Ltd.), 27 June 1986 (27. 06. 86), (Family: none) | 1 - 5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| May 25, 1990 (25. 05. 90) | June 11, 1990 (11. 06. 90) |
| **International Searching Authority** | **Signature of Authorized Officer** |
| Japanese Patent Office | |